(19) Europäisches Patentamt
European Patent Office
Office européen des brevets

(11) Publication number: 0 120 575
B1

# (12) EUROPEAN PATENT SPECIFICATION

(45) Date of publication of patent specification: 14.06.89

(51) Int. Cl.$^4$: C 07 D 213/61, C 07 D 213/84, C 07 C 17/20, C 07 B 39/00

(21) Application number: 84300911.9

(22) Date of filing: 14.02.84

## (54) Organic fluorine compounds.

(30) Priority: 18.02.83 JP 24848/83
31.10.83 JP 202590/83
24.11.83 JP 219473/83
24.11.83 JP 219474/83

(43) Date of publication of application:
03.10.84 Bulletin 84/40

(45) Publication of the grant of the patent:
14.06.89 Bulletin 89/24

(84) Designated Contracting States:
CH DE FR GB LI

(56) References cited:
EP-A-0 003 344
FR-A-2 096 028
GB-A-1 340 421
TETRAHEDRON LETTERS, no. 16, 1978, pages 1429-1432, Pergamon Press, GB H. BÖHME et al.: "Synthese und Eigenschaften von Carbimidoylfluoriden"

The file contains technical information submitted after the application was filed and not included in this specification

(73) Proprietor: Nippon Shokubai Kagaku Kogyo Co., Ltd
1, 5-chome, Koraibashi Higashi-ku
Osaka-shi Osaka-fu 541 (JP)

(72) Inventor: Kaieda, Osamu
406-59, Todaiji Shimamoto-Cho
Mishima-gun Osaka-fu (JP)
Inventor: Awashima, Masaru
1-40, Shimizu
Suita-shi Osaka-fu (JP)
Inventor: Okitaka, Isao
11-15, 3-chome Shimoshinjo
Higashiyodogawa-ku Osaka-shi Osaka-fu (JP)
Inventor: Nakamura, Toshiaki
5-15, 3-chome, Fukaeminami Higashinari-ku
Osaka-shi Osaka-fu (JP)

(74) Representative: SERJEANTS et al
25, The Crescent King Street
Leicester, LE1 6RX (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 120 575 B1

**Description**

Technical Field

The invention relates to a method of manufacturing aromatic fluorine compounds from corresponding chloro- or bromo-compounds by a halogen exchange reaction with a fluorinating agent.

Background Art

Halogen exchange is a reaction of an alkali metal fluoride with an aromatic halide to substitute a fluorine atom for the other halogen atom, see for example, Ishikawa: Journal of Synthetic Organic Chemistry, Japan, Vol. 25, page 808 (1967) and M. Hudlicky: Chemistry of Organic Fluorine Compounds, page 112 (1976), John Wiley & Sons Press.

FR—A—2096028 describes such an exchange in chloropyridines. Benzonitrile is mentioned as a suitable solvent, but in all the Examples other solvents are used, and the suitability of benzonitrile is not confirmed. Operation is always under reflux, which means atmospheric or normal pressure. In GB—A—1340421, sodium fluoride is used as the fluorinating agent. Again benzonitrile is mentioned but not actually used as a solvent, and operation is under reflux. In EP—A—0003344 chlorobenzenes which are nitro- or cyano- substituted are similarly treated, but again the use of benzonitrile is not actually confirmed, and operation is always under normal pressure, althouth the possibility of using superatmospheric pressure is stated.

The Invention

According to the invention the aromatic chlorine- or bromine- compound is treated with a fluorinating agent using benzonitrile as a solvent in the absence of an initiator and at from 190° to 400°C under at least spontaneously generated pressure.

Benzonitrile is thermally stable under these conditions. It does not undergo a secondary reaction with the reactants or with the product of the main reaction. The reaction proceeds quickly and has a good yield. The reaction temperature can easily be controlled. A large amount of carbonized materials is not produced.

Benzonitrile has only a slight ability to dissolve inorganic salts at temperatures below its boiling point. At temperatures exceeding the boiling point, the solubility of the fluorinating agent such as potassium fluoride is sharply increased. The reaction proceeds advantageously when it is performed at from 230° to 360°C. The high yield may be ascribed to the advantageously high operating temperature and the solubility of the fluorinting agent. Substitution of halogens at the meta positions can be effected.

As the starting material, there may be used a chloro- or bromo- compound in which fluorine has already been substituted for some of the chlorine or bromine atoms. Suitable compounds are of the general formula I.

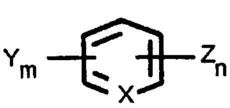
(I)

wherein X is N, CY or CZ, Y is Cl or Br, Z is F, CN or $NO_2$, m is an integer from 1 to 5, n an integer from 0 to 4, and the sum of m and n is 5. Those in which X is CCN or N are particularly advantageous.

Typical examples include polychlorobenzenes, 3,5-dichloro-2,4,6-trifluorbenzonitrile, 3-chloro-2,4,5,6-tetrafluorobenzonitrile, pentachlorobenzonitrile, tetrachlorophthalonitrile, 5-chloro-2,4,6-trifluoroisophthalonitrile, tetrachloroisophthalonitrile, tetrachloroterephthalonitrile, 3,5-dichloro-2,4,6-trifluoropyridine, pentachloropyridine, 5-chloro-2,4,6-trifluoropyridine-3-nitrile, 2,4,5,6-tetrachloropyridine-3-nitrile, 2,3,5,6-tetrachloropyridine-4-nitrile, 3,4,5,6,-tetrachloropyridine-2-nitrile and such compounds having a bromine tom substituted for the chlorine atoms therein.

Examples of suitable fluorinating agents include alkali metal fluorides such as cesium fluoride, potassium fluoride, sodium fluoride and lithium fluoride, and alkaline earth metal fluorides such as calcium fluoride, barium fluoride and magnesium fluoride. Fluorides of other metals such as antimony fluoride may sometimes be used.

Generally speking, the fluorinating agent is used in an amount at least equivalent to the chlorine or bromine atom to be substituted. In the case of an alkali metal fluoride, the amount of the fluorinating agent advantageously used is from 1 to 2 mols relative to the chlorine or bromine atoms in the organic compound treated.

The pressure in the reaction system may be increased by addition of an inert gas such as air. Generally the reaction is carried out under a pressure of from 0 to 30 $kg/cm^2$ (gauge) (0.101 to 3.043 MPa), preferably from 1.5 to 22 $kg/cm^2$ (gauge) (0.248 to 2.259 MPa).

The optimum reaction temperature depends on the starting material. When pentachlorobenzonitrile is used, the temperature is preferably 270° to 400°C (4 to 30 $kg/cm^2$·) (0.494 to 3.043 MPa), more preferably from 300°C to 350°C (7 to 20 $kg/cm^2$·G) (0.778 to 2.063 MPa). In the case of the tetrachlorophthalonitrile, the temperature is preferably from 190°C (0 to 11 $kg/cm^2$·) (0/101 to 7.180 MPa). In the case of tetrachloroisophthalonitrile, the temperature is preferably from 250° to 350°C (2.5 to 18 $kg/cm^2$·) (0.346 to 1.867 MPa). In the case of tetrachloroterephthalonitrile, the temperature is preferably from 210° to 330°C (0.7 to 15 $kg/cm^2$·G) (0.170 to 1.572 MPa). When a pentachloropyridine is used, the temperature is

preferably from 300° to 400°C (7 to 30 kg/cm$^2$·G) (0.788 to 3.043 MPa), more preferably from 330° to 380°C (10 to 26 kg/cm$^2$G) (1.082 to 2.651 MPa).

The reaction time is generally 2 to 48 hours although it depends on the temperature and the starting material. The chloro- or bromo-organic compound is used in an amouunt of 5 to 50 parts by weight, preferably from 20 to 40 parts by weight, based on 100 parts by weight of benzonitrile.

When the reaction temperature is low and the reaction time is short, there may be partly formed a reaction product in which the chlorine or bromine has not been completely substituted. Where the compounds aimed at by the reaction has a lower boiling point such as the boiling point of pentafluorobenzonitrile (161°C under 760 mmHg (0.101 MPa)) or that of pentafluoropyridine (84°C under 760 mmHg (0.101 MPa)) than the boiling point of benzonitrile (191°C under 760 mmHg (0.101 MPa)), the compound produced can be separated by stripping. In the kettle, a chlorine- or bromine-containing fluorine compound of a high boiling point remains dissolved in the benzonitrile. The benzonitrile solvent thus remaining in the kettle may be recovered and put to use again in the reaction. In the next reaction, the chlorine- or bromine-containing fluorine compound contained therein an unaltered intermediate is readily converted into the compound aimed at by the main reaction. The reuse of the recovered benzonitrile solution contributes to increasing the yield of the product.

Bezonitrile lacks hygroscopicity and, therefore, is easily separated from the reaction product. Potassium fluoride (fluorinating agent) has high hygroscopicity, and so may require to be combined with benzene or toluene and distilled to remove water in the form of azeotrope in advance of the reaction.

A phase transfer catalyst in the reaction system may prove advantageous. When the reaction system contains the phase transfer catalyst, there is an advantage that the reaction velocity can be increased and the reaction time shotened. As the phase transfer catalyst, a crown compound such as dibenzo-18-crown-6-ether or polyethylene glycol having a molecular weight of 300 to 600 can be used. The amount of the phase transfer catalyst to be advantageously used is from 0.01 to 0.25 mol, preferably 0.05 to 0.20 mol, per mol of the organic compound raw material.

Typical examples of fluorine compounds which can be manufactured according to the invention include polyfluorobenzenes, 3,5-difluoro-2,4,6-trichlorobenzonitrile, pentafluorobenzonitrile, tetrafluoro-phthalonitrile, 5-chloro-2,4,6-trifluoroisophthalonitrile, tetrafluoroterephthalonitrile, tetrafluoroterephthalonitrile, 3,5,-dichloro-2,4,6-trifluoropyridine, pentafluoropyridine, 5-chloro-2,4,6-trifluoropyridine-3-nitrile, 2,3,5,6-tetrafluoropyridine-4-nitrile and 3,4,5,6-tetrafluoropyridine-2-nitrile, which are useful compounds as intermediates for the synthesis of agricultural pesticides, medicines and dyestuffs.

Benzonitrile can be easily isolated from the reaction product by distillation and can be reused.

## EXAMPLES

### Example 1

An autoclave made of stainless steel and having an inner volume of 500 cc was charged with 200 g of benzonitrile, 60.0 g (0.218 mol) of pentachlorobenzonitrile and 69.7 g (1.20 mols) of finely divided dry potassium fluoride. After the air in the vessel was displaced with nitrogen gas, the contents, of the vessel were stirred at 315°C (under 12.5 kg/cm$^2$·G (1.327 MPa)) for 18 hours. After the reaction terminated, the reaction mixture was cooled to room temperature and the potassium chloride and the unaltered potassium fluoride suspended therein were removed by filtration. When the benzonitrile solution remaining as the mother liquid in the vessel was analyzed by the internal standard method with a gas chromatography using 2 m of a packing agent (Apiezon® Grease M) at a column temperature of 150°C, it was found to contain 80.5 mol% of pentafluorobenzonitrile and 1.2 mol% of 3,5-dichloro-2,4,6-trifluorobenzonitrile based on the amount of pentachlorobenzonitrile first placed in the autoclave. When the mother liquid was analyzed by gas chromatography using 2 m of a packing agent (Thermon® 1000) at a column temperature of 90°C, it was found to contain 4.3 mol% of 3-chloro-2,4,5,6-tetrafluorobenzonitrile based on the amount of pentachlorobenzonitrile first placed in the autoclave. One half of the mother liquid was accurately weighed out and treated with a precision fractional distilliation unit, to obtain 15.9 g (75.5 mol% in yield) of pentafluorobenzonitrile (as a fraction at 160° to 163°C under normal pressure) as the product aimed at by the reaction. When this fraction was analyzed by gas chromtography, virtually no discernible peak of any substance other than pentafluorobenzonitrile was detected.

### Example 2

An autoclave having an inner volume of 500 cc was charged with the same raw materials as used in Example 1, except that 11.5 g (0.0319 mol) of dibenzo-18-crown-6-ether was dissolved in benzonitrile. The contents of the vessel were stirred at 300°C (under 10.0 kg/cm$^2$·G (1.082 MPa)) for seven hours. After the reaction was terminated, the reaction mixture was treated by following the procedure of Example 1. When the mother liquid consequently obtained was analyzed by gas chromatography, it was found to contain 66.5 mol% of pentaflurobenzonitrile, 15.4 mol% of 3-chloro-2,4,5,6-tetrafluorobenzonitrile and 5.8 mol% of 3,5-dichloro-2,4,6-trifluorobenzonitrile based on the amount of pentachlorobenzonitrile first placed in the vessel.

Example 3

An autoclave having an inner volume of 500 cc was charged with the same raw materials as used in Example 1. The contents of the vessel were stirred at 350°C (under 17.0 kg/cm$^2$·G (1.768 MPa)) for four hours. After the reaction was terminated, the reaction mixture was treated by following the procedure of Example 1. When the mother liquid consequently obtained was analyzed by gas chromatography it was found to contain 72.5 mol% of pentaflurobenzonitrile based on the amount of pentachlorobenzonitrile first placed in the vessel.

Example 4

An autoclave made of stainless steel and having an inner volume of 100 cc was charged with 40 g of benzonitrile, 16 g (0.0322 mol) of pentabromobenzonitrile and 10.3 (0.177 mol) of finely divided dry potassium fluoride. After the air in the reaction vessel was displaced with nitrogen gas, the contents of the vessel were stirred at 300°C (under 9.5 kg/cm$^2$·G (1.062 MPa)) for 20 hours. After the reaction was terminated, the reaction mixture was treated by following the procedure of Example 1. When the mother liquid consequently obtained was analyzed by gas chromatography, it was found to contain 71.9 mol% of pentafluorobenzonitrile based on the amount of pentabromobenzonitrile first placed in the reaction vessel.

Example 5

An autoclave made of stainless steel and having an inner volume of 500 cc was charged with 200 g of benzonitrile, 80.0 g (0.291 mol) of pentachlorobenzonitrile and 65.9 g (1.135 mol) of finely divided dry potassium fluoride. After the air in the reaction vessel was displaced with nitrogen gas, the contents of the vessel were stirred at 270°C (under 7.0 kg/cm$^2$·G (0.788 MPa)) for four hours. After the reaction was terminated, the reaction solution was separated from potassium chloride and unaltered potassium fluoride by the use of a rotary evaporator under the final conditions of 180°C of external temperature and 20 Torrs (2666 Pa) of vacuum. By treating the reaction solution with a precision fractional distilation device, there was recovered 56.8 g of 3,5-dichloro-2,4,6-trifluorobenzonitrile (a fracton at 222° to 224°C under normal pressure) (86.3% in yield based on the amount of pentafluorobenzonitrile first placed in the reaction vessel, 98.2% in purity).

Example 6

An autoclave made of stainless steel and having an inner volume of 500 cc was charged with 200 g of benzonitrile, 80.0 g (0.354 mol) of 3,5-dichloro-2,4,6-trifluorobenzonitrile and 45.2 (0.779 mol)) of finely divided dry potassium fluoride. After the air in the reaction vessel was displaced with nitrogen gas, the contents, of the vessel were stirred at 330°C (under 14.0 kg/cm$^2$·G (1.474 MPa)) for 12 hours. After the reaction was terminated, the reaction solution was separated from potassium chloride and unaltered potassium fluoride with a rotary evaporator at 150° to 180°C under a vacuum. When the separated solution was analyzed by gas chromatography using 2 m of packing agent (Thermon® 1000) at a column temperature of 60°C, it was found to contain 96.8 mol% of pentachlorobenzonitrile based on the amount of 3,5-dichloro-2,4,6-trifluorobenzonitrile first placed in the vessel.

By treating the separated solution with a precision fractional distillation device, there was recovered 62.8 g of pentafluorobenzonitrile as the product aimed at by the reaction. When this fraction was analyzed by gas chromatography, virtually no discernible peak of any substance other than pentafluorobenzonitrile was detected.

Example 7

An autoclave made of stainless steel and having an inner volume of 500 cc was charged with 200 g of benzonitrile, 80.0 g (0.301 mol) of tetrachloroisophthalonitrile and 83.9 (1.445 mol)) of finely divided dry potassium fluoride. After the air in the reaction vessel was displaced with nitrogen gas, the contents, of the vessel were stirred at 320°C (under 13.0 kg/cm$^2$·G (1.376 MPa)) for 18 hours. After the reaction was terminated, the reaction mixture thus produced was cooled to room temperature and potassium chloride and unaltered potassium fluoride suspended therein were removed from the reaction mixture by filtration. The benzonitrile solution which remained as the other liquid was analyzed by the inner standard method using a gas chromatography using 1 m of a packing agent (SE 52) at a column temperature of 60°C. Consequently, there was obtained 90.5 m% of tetrafluoroisophthalonitrile based on the amount of tetrachloroisophthalonitrile first placed in the reaction vessel. In the chart of the analyses, virtually, no discernible peak of any other substance such as unsubstituted isophthalonitrile was detected. By the gas chromatographic mass spectrometry (70 eV/ m/e = 200, 131, 100, 31), the peak was confirmed to be that of tetrafluoroisophthalonitrile. By expelling the solvent benzonitrile from the aforementioned mother liquid through vacuum distillation, there was recovered 52.5 g of tetrafluoroisophthalonitrile in the form of crystals (M.P.; 73° to 76° C). By elementary analysis, the crystals were found to consist of 48.0% of carbon, 38.2% of fluorine, and 13.7% of nitrogen (theoretically 48% of carbon, 38% of fluorine and 14% of nitrogen).

Example 8

An autoclave having an inner volume of 500 cc was charged with the same raw materials as used in Example 7, except that 5.8 g (0.016 mol) of dibenzo-18-crown-6-ether was dissolved in benzonitrile. The contents of the vessel were stirred at 280°C (under 7.0 kg/cm$^2$·G (0.788 MPa)) for 10 hours. After the reaction terminated, the resultant reaction mixture was treated by following the procedure of Example 7. When the mother liquid consequently obtained was analyzed by gas chromatography it was found to

contain 70.2 mol% of tetrafluorobenzonitrile and 18.7 mol% of 5-chloro-2,4,6-trifluoroisophthalonitrile based on the amount of tetrachloroisophthalonitrile first placed in the vessel.

Example 9

An autoclave having an inner volume of 500 cc was charged with the same raw materials as used in Example 7, except that tetrachloroterephthalonitrile was used as the starting material in the place of tetrachloroisphthalonitrile. The contents of the vessel were stirred at 270°C (under 6.0 kg/cm$^2$·G (0.690 MPa)) for 12 hours. After the reaction terminated, the resultant reaction mixture was treated by following the procedure of Example 7. When the mother liquid consequently obtained was analyzed by gas chromatography, it was found to contain 92.2 mol% of tetrafluoroterephthalonitrile based on the amount of tetrachloroterephthalonitrile first placed in the vessel. By expelling the solvent benzonitrile from this mother liquid through vacuum distillation, there was obtained tetrafluoroterephthalonitrile in the form of crystals (M.P.; 195°—197°C).

Example 10

An autoclave made of stainless steel and having an inner volume of 500 cc was charged with 200 g of benzonitrile, 80.0 g (0.301 mol) of tetrachloroorthophthalonitrile, and 83.9 g (1.444 mol) of finely divided dry potassium fluoride. After the air in the reaction vessel was displaced with nitrogen gas, the contents, of the vessel were stirred at 230°C (under 2.0 kg/cm$^2$·G (0.297 MPa)) for 10 hours. After the reaction terminated, the resultant reaction mixture was cooled to room temperature and potassium chloride and unaltered potassium fluoride suspended in the reaction mixture were removed by filtration. When the benzonitrile solution remaining as the mother liquid was analyzed by gas chromatography using 1 ml of packing agent (SE 52) at a column temperture of 60°C, it was found to contain 87.7 mol% of tetrafluoroorthophthalonitrile based on the amount of tetrachloroorthophthalonitrile first placed in the reaction vessel.

By expelling benzonitrile from the mother liquid through vacuum distillation, there was obtained crystals of tetrafluoroorthophthalonitrile (M.P.; 86°—87°C) which solidified at room temperature.

Example 11

An autoclave made of stainless steel and having an inner volume of 500 cc was charged with 200 g of benzonitrile, 50.0 g (0.200 mol) of pentachloropyridine, and 69.7 g (1.20 mols) of finely divided dry potassium fluoride. After the air in the vessel was displaced with nitrogen gas, the contents, of the vessel were stirred at 365°C (under 20.5 kg/cm$^2$·G (2.112 MPa)) for 30 hours. After the reaction terminated, the reaction solution was freed from potassium chloride and the unaltered potassium fluoride with a rotary evaporator under the final conditions of 160°C of external temperature and 20 Torrs (2666 Pa) of vacuum. When the separated solution was analyzed by gas chromatography using 2 m of a packing agent (Thermon® 1000) at a column temperature of 60°C, it was found to contain 54.2 mol% of pentafluoropyride, 9.3 mol% of 3-chloro-2,4,5,6-tetrafluoropyridine and 26.6 mol% of 3,5-dichloro-2,4,6-trifluoropyridine respectively based on the amount of pentachloropyridine first placed in the vessel.

By treating the separated solution with a precision fractional distilliation device, there was obtained 17.9 g of pentafluoropyridine (fraction at 83° to 85°C under normal pressure) as the product aimed at. When this fraction was analyzed by gas chromatography, virtually no discernible peak of any substance other than pentafluoropyridine was detected.

Example 12

An autoclave having an inner volume of 500 cc was charged with the same raw materials as used in Example 11, except that 11.5 g (0.0319 mol) of dibenzo-18-crown-6-ether was dissolved in benzonitrile. The contents of the vessel were stirred at 330°C (under 15.0 kg/cm$^2$·G (1.572 MPa)) for 24 hours. After the reaction terminated, the resultant reaction mixture was treated by following the procedure of Example 11. When the mother liquid consequently obtained was analyzed by gas chromatography it was found to contain 25.7 mol% of pentafluoropyridine, 13.2 mol% of 3-chloro-2,4,5,6-tetrafluoropyridine and 54.3 mol% of 3,5-dichloro-2,4,6-trifluoropyridine respectively based on the amount of pentachloropyridine first placed in the vessel.

Example 13

An autoclave made of stainless steel and having an inner volume of 200 cc was charged with 100 g of benzonitrile, 40 g (0.165 mol) of 2,4,5,6-tetrachloropyridine-3-nitrile and 46 g (0.790 mol) of finely divided dry potassium fluoride. After the air in the vessel was displaced with nitrogen gas, the contents, of the vessel were stirred at 320°C (under 13.5 kg/cm$^2$·G (1.425 MPa)) for 10 hours. After the reaction terminated, the resultant reaction solution was freed from potassium chloride and the unaltered potassium fluoride by the use of a rotary evaporator under the final conditions of 160°C of outer temperature and 20 Torrs (2666 Pa) of vacuum. When the separated solution was analyzed by gas chromatography using 1 m of a packing agent (SE 52) at a column temperature of 60°C, it was found to contain 79.4 mol% of 2,4,5,6-tetrafluoropyridine-3-nitrile and 5.1 mol% of 5-chloro-2,4,6-trifluoropyridine-3-nitrile respectively based on the amount of 2,4,5,6-tetrachloropyridine-3-nitrile first placed in the vessel.

When the separated solution was treated with a precision fractional distillation device, there was recovered 22 g 2,4,5,6-tetrafluoropyridine-3-nitrile (fraction at 165°—166°C under normal pressure) as the

product aimed at. When this fraction was analyzed by gas chromatography, virtually no discernible peak of any substance other than 2,4,5,6-tetrafluoropyridine-3-nitrile was detected.

Example 14

An autoclave made of stainless steel and having an inner volume of 100 cc was charged with 40 g of benzonitrile, 8.0 g (0.028 mol) of hexachlorobenzene and 14.6 g (0.252 mol) of finely divided dry potassium fluoride. After the air in the reaction vessel was displaced with nitrogen gas, the contents of the vessel were stirred at 350°C (under 16.0 $kg/cm^2 \cdot G$ (1.670 MPa)) for 30 hours. After the reaction terminated, the reaction mixture was cooled at room temperature and potassium chloride and unaltered potassium fluoride were removed by filtration. When the benzonitrile solution remaining as the mother liquid was analyzed by gas chromatography using 2 m of a packing agent (Thermon ® 1000) at a column temperature of 60° to 120°C (gradual elevation), it was found to contain 20.3 mol% of hexafluorobenzene, 57.1 mol% of monochloropentafluorobenzene and 10.6 mol% of dichlorotetrafluorobenzene respectively based on the hexachlorobenzene first placed in the vessel.

**Claims:**

1. A method of manufacturing an aromatic fluorine compound from a corresponding chloro- or bromo-compound having a substituent on each carbon atom by a halogen exchange reaction with an alkali metal fluoride or an alkaline earth metal fluoride fluorinating agent *characterized in that* the aromatic chlorine- or bromine-compound is treated with a fluorinating agent using benzonitrile as a solvent in the absence of an initiator and at from 190°C to 400°C under at least spontaneously generated pressure.

2. A method according to claim 1 characterized in that the fluorinating agent is potassium fluoride.

3. A method according to claim 1 or claim 2 characterized in that the chloro- or bromo- compound is used in an amount of from 5 to 50 parts by weight based on 100 parts by weight of benzonitrile.

4. A method according to any preceding claim characterized in that the fluorinating agent is an alkali metal fluoride in an amount of from 1 to 2 mols relative to the chlorine or bromine atoms in the organic compound treated.

5. A method according to any preceding claim characterized in that it is carried out in the presence of a phase transfer catalyst.

6. A method according to claim 5 characterized in that the phase transfer catalyst is used in an amount of from 0.01 to 0.25 mol per mol of the organic compound treated.

**Patentansprüche**

1. Verfahren zur Herstellung einer aromatischenm Fluorverbindung aus einer entsprechenden Chlor- oder Bromverbindung, die infolge einer Halogenaustauschreaktion mit einem Alkalimetallfluorid- oder einem Erdalkalimetallfluorid-Fluorinationsagens an jedem Kohleatom ein Ersatzatom aufweist, *dadurch gekennzeichnet daß* die aromtische Chlor- oder Bromverbindung mit einem Fluorinationsagens — wobei in Abwesenheit eines Initiators Benzonitril als Lösungsmittel verwendet wird — und bei einer Temperatur zwischen 190° und 400°c unter mindestens spontan erzeugtem Druck behandelt wird.

2. Verfahren gemäß Anspruch 1, dadurch gekennzeichnet daß als Fluorinationsagens Kaliumfluorid verwendet wird.

3. Verfahren gemäß Anspruch 1 oder Anspruch 2, dadurch gekennzeichnet daß für 100 Gewichtsteile Benzonitril 5 bis 50 Gewichtsteile Chlor- oder Bromverbindung eingesetzt werden.

4. Verfahren gemäß einem der vorhergehenden Ansprüche, dadurch gekennzeichnet daß als Fluorinationsagens ein Alkalimetallfluorid in Mengen von 1 bis 2 mol, bezogen auf die Chlor- oder Bromatome in der behandelten organischen Verbindung, verwendet wird.

5. Verfahren gemäß einem der vorhergehenden Ansprüche, dadurch gekennzeichnet daß das Verfahren in Gegenwart eines Phasentransfer-Katalysators durchgeführt wird.

6. Verfahren gemäß Anspruch 5 dadurch gekennzeichnet daß 0,01 bis 0,25 mol Phasentransfer-Katalysator pro mol der behandelten organischen Verbindung verwendet werden.

**Revendications**

1. Une méthode de fabrication d'un composé aromatique du fluor tiré d'un compose de chlore ou bromure ayant un replaçant sur chaque stome de carbone par une réaction d'échange halogène avec une fluorure métallique alcaline ou un agent fluorisant de fluorure métallique de terre alcalin, caractérisée par le fait que le composant de chlore ou brome aromatique est traité avec un agent fluorant utilisant du benzonitrile comme solvent en l'absence d'un initiateur et à une température de 190° à 400°C sous pression au moins produite spontanément.

2. Une méthode, selon la revendication 1, caractérisée par le fait que l'agent fluorant est du fluorure de potassium.

3. Une méthode, selon la revendication 1 ou la revendication 2, caractérisée par le fait que le

composant de chlore ou bromure est utilisé en une quantité de 5 à 50 parts par poids basé sur 100 parts par pods de benzonitrile.

4. Une méthode, selon toute revendication précédente, caractérisée par le fait que l'agent fluorisant est une fluorure metallique alcaline en quantité de 1 à 2 mol se rapportant aux atomes de chlore ou brome dans le composant organique traité.

5. Une méthode, selon toute revendication précédente, caractérisée par le fait qu'elle est realisée en présence d'unn catalyseur de transfert de phase.

6. Une méthode, selon la revendication 5, caractérisée par le fait que le catalyseur de transfert de phase est utilisé en quantité de 0.01 à 0.25 mol par mol de composant organique traité.

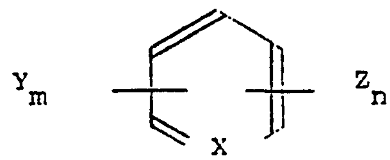

(I)